(19) Europäisches Patentamt
European Patent Office
Office européen des brevets

(11) Publication number: 0 114 316 B1

# (12) EUROPEAN PATENT SPECIFICATION

(45) Date of publication of patent specification: 30.09.87

(51) Int. Cl.$^4$: G 01 N 33/493, G 01 N 33/52, G 01 N 9/00

(21) Application number: 83112689.1

(22) Date of filing: 16.12.83

## (54) Test means, test device and method for determining the ionic strength or specific gravity of a liquid sample.

(30) Priority: 27.12.82 US 453874
27.12.82 US 453297

(43) Date of publication of application:
01.08.84 Bulletin 84/31

(45) Publication of the grant of the patent:
30.09.87 Bulletin 87/40

(84) Designated Contracting States:
CH DE FR GB IT LI NL SE

(56) References cited:
EP-A-0 023 631
US-A-2 984 639
US-A-3 325 402
US-A-3 429 963
US-A-4 318 709
US-A-4 320 040

CHEMICAL ABSTRACTS, vol. 84, no. 22, 31st May 1976, page 16, no. 151195c, Columbus, Ohio, USA; E. TSUCHIDA et al.: "Gel formation through interpolymer interaction of poly(methacrylic acid)-poly(vinylbenzyltrimethylammonium chloride"

(73) Proprietor: MILES LABORATORIES, INC.
1127 Myrtle Street
Elkhart Indiana 46514 (US)

(72) Inventor: Wang, Joseph Y.
1501 Locust Street Apt. 207
Elkhart IN 46514 (US)

(74) Representative: Dänner, Klaus, Dr. et al
c/o Bayer AG Konzernverwaltung RP
Patentabteilung
D-5090 Leverkusen 1 Bayerwerk (DE)

(56) References cited:
CHEMICAL ABSTRACTS, vol. 95, no. 18, 2nd November 1981, page 11, no. 151300k, Columbus, Ohio, USA; B. PHILIPP et al.: "Polysalt formation with poly(carboxylic acid)s"

CHEMICAL ABSTRACTS, vol. 81, no. 23, 9th December 1974, page 24, no. 153025j, Columbus, Ohio, USA; ZH.G. GULYAEVA et al.: "Polyelectrolyte complexes consisting of polymeric quaternary ammonium salts"

CHEMICAL ABSTRACTS, vol. 95, no. 22, 30th November 1981, page 12, no. 187841b, Columbus, Ohio, USA;

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 114 316 B1

## Description

Contents

1. Introduction
 1.1 Applications of the Invention
 1.2 Relationship Between Specific Gravity and Ionic Strength

2. Background of the Invention
3. Summary of the Invention
4. Brief Description of the Drawings
5. Definitions
6. Polyelectrolytes and Their Salts
 6.1 Weakly Acidic Polyelectrplytes
 6.2 Quaternary Ammonium Ions
 6.3 Preparation of the Polyelectrolyte Salt

7. pH Indicator Means
8. The Test Device
 8.1 The Carrier Matrix
 8.2 Incorporation of the Matrix with the Composition
 8.3 Preparation of a Dip-and-Read Device

9. Reference Standard
10. Examples
 10.1 Preparation of the Polyelectrolyte Salt
 10.2 Preparation of Test Devices
 10.3 Evaluation of the Test Device

1. Introduction

The present invention relates to a test means for determining the ionic strength or specific gravity of a test sample. Furthermore, test device and method are disclosed for making this determination in an aqueous test sample. These aspects of the invention provide a simple, facile method for analyzing ionic strength or specific gravity whereby results are available to the assayist momentarily after merely contacting a test sample solution with the test means or device. There is no need for such cumbersome apparatuses and procedures as hydrometers, urinometers, gravimeters, calibration, the cleaning of equipment, or other trappings of prior procedures.

1.1 Applications of the Invention

The determination of the specific gravity of a liquid has application in numerous arts. Such seemingly unrelated disciplines as brewing, urinalysis, water purification, and the preparation of drinking water aboard a ship at sea all involve the measurement of specific gravity. Needless to say, a quick, facile method for determining this solution property would greatly enhance the state of these technologies, as well as any others where rapid, accurate determination of specific gravity would be beneficial. Thus, for example, if a medical laboratory technician could accurately measure the specific gravity of a urine sample in a matter of seconds, not only would such rapid results aid the physician in diagnosis, but also laboratory efficiency would increase manyfold.

Although the present invention lends itself to a vast range of applications, for purposes of clarity this discussion will be couched largely in terms of the determination of the ionic strength or specific gravity of urine. Applications to other disciplines will become apparent from an understanding of how this invention relates to urinalysis.

The determination of urine specific gravity is of considerable value in the understanding and clinical management of electrolyte disturbances. Hence, complete urinalysis should, and usually does, include a specific gravity determination. Generally, such a determination would include the measurement of specific gravity directly with a suitable device, but equally useful is the measurement of some related property, such as osmolality or ionic strength, which can then be referred back to corresponding specific gravity values.

1.2 Relationship Between Specific Gravity and Ionic Strength

Specific gravity is a dimensionless term and relates, in the case of a solution, to the ratio of the weight of a certain volume of the solution at a given temperature to that of an equal volume of water, also at some specified temperature. For solutions such as urine, the specific gravity is a function of the number, density, ionic charge, and weight of the various species of dissolved solutes.

The term "ionic strength" refers to the mathematical relationship between the number of different

kinds of ionic species in a particular solution and their respective charges. Thus, ionic strength μ is represented mathematically by the formula.

$$\mu = \tfrac{1}{2}\sum_i c_i z_i^2 \qquad (1)$$

in which c is the molal concentration of a particular ionic species and z the absolute value of its charge. The sum Σ is taken over all the different kinds of ions in solution.

The relationship between ionic strength and specific gravity has a definable mathematical correlation. In the case of dilute NaCl, for example, in which the solution has a molal concentration of c, equation (1) reduces to

$$\mu = \tfrac{1}{2}\sum_i c_i z_i^2 \qquad (1)$$

$$= \tfrac{1}{2}\left[(C_{Na}+)(1) + (c_{Cl}-)(1)\right] \qquad (2)$$

$$\mu = c \qquad (3)$$

Moreover, it is known that the relationship between molarity c and molality M of a given solution is

$$c = \frac{M}{\rho}$$

where ρ is the density of the solvent. Substituting c from equation (3) into equation (4) yields the relationship between strength and molarity.

$$\mu = \frac{M}{\rho} \qquad (5)$$

For dilute NaCl, it has been found experimentally that the following relationships between molar concentration (M) and specific gravity (SG) exist:

| SG | ΔSG | M NaCl | ΔM |
|---|---|---|---|
| 1.005 | 0.120 | 0.240 | 0.120 |
| 1.010 | 0.005 | 0.360 | 0.120 |
| 1.015 | 0.005 | 0.488 | 0.128 |
| 1.020 | 0.005 | 0.613 | 0.125 |
| 1.025 | 0.005 | | |

The data shows that for every incremental increase of 0.12 M in NaCl concentration, a corresponding change in SG of 0.005 occurs. Using this relationship, SG can be defined mathematically as

$$SG = 1 + \frac{0.005M}{0.12} \qquad (6)$$

Substituting (5) into (6) we have

$$SG = 1 + \frac{0.005\mu\rho}{0.12} \qquad (7)$$

Where the solvent is water, ρ = 1 and equation (7) reduces to

$$SG = 1 + \frac{0.005\mu}{0.12} \qquad (8)$$

2. Background of the Invention

Prior to the present invention, most methods for determining specific gravity utilized hydrometers, urinometers, pycnometers and gravimeters. Athough these prior art procedures are satisfactorily sensitive in most cases, they involve fragile, bulky instruments which must be constantly cleaned, maintained, and calibrated in order to continuously assure their reliability. In addition, there are many inconveniences associated with the mechanics of using these instruments. There may be a difficulty in reading the miniscus. Froth or bubbles on the liquid surface may interfere with the reading. There is a tendency for urinometers to adhere to the sides of the vessel containing the liquid sample. In the case of urine, the sample quantity is frequently inadequate for accommodating one of the aforementioned devices.

A recent breakthrough in which all of the above disadvantages have been virtually eliminated, and which affords rapid osmolality (*ergo,* specific gravity) determination, is disclosed in U.S. Patent No. 4,015,462, issued to Greyson, *et al.,* and assigned to the present assignee. This patent describes an invention in which a carrier matrix is incorporated with osmotically fragile microcapsules, the walls of which are composed of a semi-permeable membrane material. Encapsulated inside the walls is a solution containing a coloring substance. When the capsules come in contact with a solution having a lower osmolality than that within the capsules, an osmotic gradient occurs across the capsule walls in the direction of the lower osmolality, thereby increasing the hydrostatic pressure within the capsules, thus causing them to swell and, ultimately, to rupture, releasing their coloured contents. The amount of color formed from this phenomenon is a function of the specific gravity of the solution.

Thus, it is seen that, besides the numerous devices which measure specific gravity directly, it is also possible to measure specific gravity using an indirect means such as the osmolality of a solution.

Yet another way of estimating specific gravity without measuring it directly involves a determination which is proportional to the ionic strength of a solution, the correlation of which parameters has already been discussed in section 1.1, *supra.* Such an approach is utilized in U.S. Patent No. 4,318,709 issued to Falb, *et al.,* and assigned to the present assignee. Since it is well known that the specific gravity of an aqueous system is greatly affected by the presence of charged species, it is possible to closely approximate the specific gravity of the respective solutions via measurements proportional to their ionic strengths, and refer those measurements to a precalibrated reference system. The Falb, *et al.,* patent makes use of such a relationship.

The Falb *et al.* patent discloses the use of weakly acidic or basic polyelectrolytes which have been at least 50% neutralized with a base (such as NaOH) or an acid (Such as HCl), respectively. Depending on the ionic strength of the test solution, an intramolecular pH change may occur in the polymer, the degree of which is a barometer of ionic strength. A pH indicator such as a pH meter or pH-sensitive compound reflects the pH change (or lack thereof) instigated by the sample ionic strength.

Both the osmolality approach and the ionic strength approach to indirectly determining specific gravity could conceivably be affected insofar as accuracy is concerned by the presence of nonionic species. However, it has been found that such nonionic constituents as glucose, protein and urea do not effectively lead to anomalous or substantially inaccurate results with the Falb, *et al.* test except at very high concentrations. See Burkhardt, *et al., Clinical Chemistry, 28,* 2068—2072 (1982).

U.S. Patent No. 4,108,727 is directed to a method for removing this potential source of inaccuracy, and discloses a device in which the specific gravity-sensitive system contains an ionizing agent capable of converting the nonionic solute to ionized species.

U.S. Patent No. 3,449,080 discusses measuring dissolved sodium or chloride ions. This reference is directed to a test device for determining the concentrations of these ions in body sweat. There is disclosed in this patent the use of ion exchange resins together with a pH indicator. Using this device, the presence of sodium or chloride ions is said to be determined through a color change in the ion exchange resin caused by the pH indicator. Whereas this reference purports to disclose a way of measuring ionic strength, it was found by the present inventors that such teachings, as set forth in the examples, were inapplicable to the measurement of specific gravity.

To summarize the present background of specific gravity measurement prior to the present invention, many methods are known for asaying that solution parameter, both direct and indirect. Direct measurement includes utilizing devices which are fragile, bulky and expensive, and which must be constantly cleaned, maintained and calibrated. Of the indirect methods, the measurement of the colligative solution property known as osmolality can provide an accurate correlation to specific gravity. In addition, the relationship between specific gravity and the ionic strength of a solution can be employed, by utilizing partially neutralized polyelectrolytes and a pH indicator. Weak polyelectrolytes are said to be useful in gauging the concentration of sodium and/or chloride ions in body sweat.

None of the prior art, however, deals with the weakly acidic polyelectrolyte quaternary ammonium salts of the present invention. These compounds enable dramatic improvements in the measurement of ionic strength, ergo specific gravity. Practice of the invention affords greater sensitivity in differentiating various specific gravity levels, as well as enhanced resistance to interference from test-to-test sample pH variations.

3. Summary of the Invention

The present invention relates to a test means, device, and method for determining the specific gravity

of an aqueous test sample. The test means for determining the ionic strength or specific gravity of an aqueous test sample, comprising a weakly acidic polyelectrolyte polymer, and a pH-indicator means capable of producing a detectable response to ion exchange between said polyelectrolyte and said sample is characterized in that said polyelectrolyte is a salt of a weakly acidic polyelectrolyte polymer wherein at least one of the carboxyl groups of the polymer is in the form of the salt of a quaternary ammonium ion having the structure

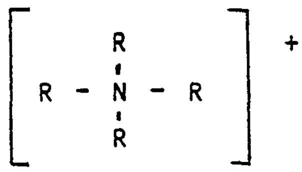

in which the R substituents, same or different, are hydrogen, 2-hydroxyethyl, benzyl, lower alkyl having one to six carbon atoms or aryl having one or more six-membered ring systems, with the proviso that at least one of R is other than hydrogen. The device of the present invention comprises a carrier matrix incorporated with the test means. The method of the present invention comprises contacting a test sample with the device or test means and observing a detectable response such as a change in color, pH or enzyme activity.

The weakly acidic polyelectrolyte polymer is at least partially, preferably at least 50 percent neutralized. Thus, one or more and, preferably, at least 50 percent of the carboxyl groups attached to the polymer backbone are present in the form of the salt of a quaternary ammonium ion. From a generic standpoint, such a polyelectrolyte salt can be thought of as having the structure

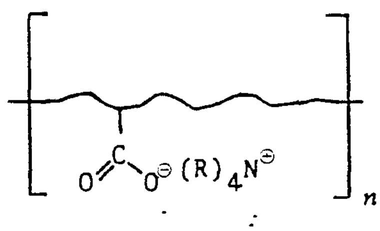

in which the wavy line represents any polymeric backbone, which may be substituted with moieties in addition to and other than, the specified carboxyl groups, and in which *n* represents the number of repeating units in the polymer chain. The R substituents of the ion $(R)_4N^+$ can be, same or different hydrogen 2-hydroxyethyl, benzyl, $C_{1-6}$, alkyl, or aryl. At least one of the R groups is other than hydrogen.

4. Brief description of the Drawings

In order to further illustrate the present invention and its advantages, the attached drawings have been provided. Figure I presents performance data from tests comparing the present invention with the prior art. It graphically portrays the surface pH developed in two pH-sensitive filter papers, one comprising the polyelectrolyte salt, the other a prior art specific gravity-sensitive system.

Figure II presents similar data in yet another fashion. This graph shows the change in pH (ΔpH) caused by various specific gravity increments in the present polyelectrolyte quaternary ammonium salts.

5. Definitions

Certain terms used in the present discussion should at this point be mentioned to assure that the reader is of the same mind as the author as to their respective meanings. Thus the following definitions are provided in order that the reader be fully apprised of the scope of the present invention, and that he be fully enabled to formulate and use it.

1. The term "lower alkyl" includes alkyl groups having one to six carbon atoms. Thus, it includes methyl, ethyl, *n*-propyl, isopropyl, n-butyl, isobutyl, *tert*-butyl and all of the pentyl and hexyl isomers. Such groups may be substituted or unsubstituted although, of course, substituents which would adversely affect the use of the invention by being reactive so as to interfere with the intended functions of its components are clearly outside the intended meaning of the term. Such interfering substituents are easily determinable at the laboratory bench through routine experimentation in keeping with the teachings of the present disclosure and the Examples.

2. As used herein, the term "aryl" relates to groups having one or more six-membered ring systems which contain the structure of benzene or other aromatic derivatives. Typical of aryl groups are phenyl ($C_6H_5$—), benzyl ($C_6H_5CH_2$—), and naphthyl. Like the lower alkyl groups, the aryl groups may be substituted or unsubstituted, provided the substituent not interfere with the intended function of the invention, i.e., the measurement of ionic strength or specific gravity.

3. As used herein, the term "quaternary ammonium salt" connotes a departure from ordinary usage of these words. In the present disclosure a quaternary ammonium salt is intended to mean a salt having the

formula $(R)N^+X^-$ in which one or more of the R substituents is hydrogen, lower alkyl or aryl, and X is halogen, in which at least one or R is other than hydrogen.

4. Likewise "quaternary ammonium hydroxide" is intended to mean a compound of the formula $(R)_4N^+OH^-$ in which at least one of the R substituents is lower alkyl or aryl.

5. "Quaternary ammonium ion" is intended to mean the positively charged ionic constituent of a quaternary ammonium salt or hydroxide. It is an ion of the formula $(R)_4N^+$ where at least one R substituent is lower alkyl or aryl.

6. "Polyelectrolyte salt" is intended to mean a weakly acidic polyelectrolyte polymer, one or more and preferably at least 50 percent of the carboxyl groups of which have been chemically combined with a quaternary ammonium ion. The salt is one in which the carboxyl proton ($H^+$) has been replaced by the quaternary ammonium ion.

6. Polyelectrolytes and their Salts

6.1 Weakly Acidic Polyelectrolytes

The salt comprises a partially neutralized weakly acidic polyelectrolyte. Numerous examples of such polymers are known in the art, their common characteristics centering about the presence of acidic pendant groups which only partially dissociate when the polymer is subjected to an aqueous environment. Most polyelectrolytes are soluble or partially soluble in water, and are readily ionizable, depending on the ionic nature of (a) the aqueous system and (b) the ionizable species on the polymer chain.

Thus a polyelectrolyte is branded weakly or strongly acidic depending on its ionic behavior in solution. Generally, polyelectrolyte which nearly completely ionizes when contacted with water, such as poly(vinylsulfonic acid) and poly(styrene sulfonic acid), are considered strong polyelectrolytes. Weakly acidic polyelectrolytes on the other hand contain weakly acidic ionizable groups, such as carboxyl groups. The charge density along the molecular chain of these polymers can be varied by varying the degree of substitution, as well as the degree of neutralization. Examples of weakly acidic polyelectrolytes which find particular applicability to the present invention are poly(acrylic acid), poly(maleic acid), maleic acid/methylvinyl ether copolymer, poly(methacrylic acid), and styrene/maleic acid copolymer. Especially suitable is a polymer known as Gantrez® AN—119, a maleic anhydride/methyl-vinyl ether copolymer marketed by General Aniline and Film Corporation.

While the composition and test means of the present invention includes weakly acidic polyelectrolytes, at least some of the functional groups of the polmer (e.g., COOH) are first partially reacted to form a salt, as specified *supra*. Thus, the polyelectrolyte salt can be prepared by at least partially titrating the polymer with a quarternary ammonium hydroxide, preferably until at least 50% of the carboxyl groups have been neutralized.

6.2 Quaternary Ammonium Ions

The quaternary ammonium ion which is included in the present invention can be obtained through well-established syntheses. Quaternary ammonium salts can be formed through reaction of alkyl or aryl halides with tertiary amines. For the purposes of the present invention, such compounds include one or more substituent groups, the remaining N-substituents being hydrogen. These salts are ionic substances and, in aqueous solution, are capable of replacing the proton ($H^+$) of a carboxyl group, thus forming a salt such as the polymer salts included in the present invention. Formation of quaternary ammonium salts is exemplified by the equation

$$(R)_3N + RX \xrightarrow[\text{heat}]{} (R)_4N^{\oplus}X^{\ominus} \qquad (9)$$

in which R is as defined, *supra*, and X is a hydrogen.

Preparation of the weakly acidic polyelectrolyte salts can ideally be performed through the use of quaternary ammonium hydroxides having the formula $(R)_4N^{\oplus}OH^{\ominus}$ in which R is as defined, *supra*. These compounds can be prepared from quaternary ammonium salts by reaction with an alkali in accordance with

$$(R)_4N^{\oplus}X^{\ominus} + MOH \leftrightarrows (R)_4N^{\oplus}OH^{\ominus} + MX \qquad (10)$$

in which X is halogen and M is an alkali metal.

An alternative method of preparation of the quaternary ammonium hydroxide is through treatment of the corresponding ammonium halide in aqueous solution with silver hydroxide in accordance with

$$(R)_4N^{\oplus}X^{\ominus} + AgOH \rightarrow (R)_4N^{\oplus}OH^{\ominus} + AgX \downarrow \qquad (11)$$

This procedure overcomes the problem of the equilibrium in (10), because of the insolubility of silver halide. Thus, if the equilibrium is shifted to the right the desired hydroxide product can then be isolated by filtering the aqueous solution followed by evaporation. The organic base can be obtained as a crystalline solid, usually a deliquescent hydrate.

Exemplary of quaternary ammonium hydroxides which produce salts with polyelectrolytes useful in the present invention are tetramethyl, tetraethyl, tetrabutyl, tributylmethyl, trimethylbutyl, trimethylphenyl, trimethylbenzyl, trimethyl-2-hydroxyethyl, trimethyl, triethyl, and tributyl ammonium hydroxides.

6.3 Preparation of the Polyelectrolyte Salt

The weakly acidic polyelectrolyte salt of the present invention may be prepared by aqueous titration of the polyelectrolyte polymer using a solution of a quaternary ammonium hydroxide. The carboxyl groups are preferably at least 50 percent neutralized. An ideal neutralization range, and that presently found most preferred in the present invention, is from 65 to 95% neutralization, 90% having thus far been found to be optimum in providing the largest separation in pH·change or other detectable response with respect to specific gravity or ionic strength.

7. pH Indicators means

Another element of the present invention is an indicator means. It can take on such diverse forms as a pH indicator compound, an enzymatic system whose enzyme/substrate function is responsive to subtle pH changes, a pH meter, and a pH-sensitive antigen/antibody system. Thus, known pH-sensitive chromogenic reagent compounds can be employed, and these can provide a change in or appearance of color, observable by the person performing the measurement, which is indicative of the ionic strength or specific gravity of the system being tested. If a chromogen is used, a reference color system can be established beforehand, so that a quick visual comparison of the composition and the reference system provides the sought-after results. Examples of chromogens suitable for use in the present invention are bromothymol blue, alizarin, bromcresol purple, phenol red and neutral red; bromothymol blue having been found to be especially suitable.

Alternatively, the indicator means can take the form of a pH meter, whereby small changes in pH (ΔpH) can be monitored directly, without resorting to visual observation of color change. One particularly suitable approach is to use the pH meter in conjunction with a surface pH electrode. The pH meter response can then be observed over various ionic strength values and a reference system can be established, a particular change in pH corresponding to a particular test sample ionic strength.

Yet another ramification of the indicator means is a pH-sensitive enzyme-based system, whereby subtle changes in pH caused by the polyelectrolyte/ionic strength interaction can trigger the onset of enzymatic activity, or which can change kinetic reaction parameters such as the $K_M$ for a particular enzymatic reaction. Thus an enzymatic system capable of providing a detectable response can be triggered to produce that response in accordance with the specific gravity or ionic strength of a test sample. For example, the enzyme chymotrypsin is known to be sensitive to pH in acting on the substrate *p*-nitrophenyl acetate to yield the yellow product, *p*-nitrophenol. The reaction rate dramatically increases from pH 6 to 8 and the appearance of *p*-nitrophenol is markedly enhanced by pH increases in that range.

Similarly, an antigen-labeled substrate can be employed. The pH dependence of antigen/antibody reactions is well known, and the indicator means of the present invention can include such a labeled substrate and the antibody for the label. Change in pH can be measured by change in substrate available for a corresponding enzymatic reaction.

8. The Test Device

The test device of the present invention comprises a suitable carrier matrix which has been incorporated with the polyelectrolyte salt and a pH-indicator compound or other pH-sensitive means, together with other inert ingredients. In an especially convenient format, a portion of the composition-bearing matrix can be mounted on one end of a plastic strip, the other end serving as a handle. Such a device can then be used to assay the ionic strength or specific gravity of a test sample merely by dipping the matrix into the sample, removing it, and observing the color of the matrix, e.g., by comparing it to a reference color chart.

8.1 The carrier matrix

The carrier matrix is usually, but not necessarily, a porous substance such as filter paper. Other art-recognized forms of carrier matrix materials are felt, porous ceramic strips, and woven or matted glass fibers (U.S. Patent No. 3,846,247). Also suggested are the use of wood, cloth, sponge materials and argillaceous substances (U.S. Patent No. 3,552,928). All such carrier matrix materials are feasible for use in the present invention, as are others. It has been found that filter paper is especially suitable.

8.2 Incorporation of the Composition with the Matrix

The method by which a reagent composition which includes the polyelectrolyte salt is incorporated with a carrier matrix is intended as broad in scope, and depends largely on the nature of the matrix. For example, where the carrier is a polymeric film, the polyelectrolyte salt and pH indicator can be cast on the film surface as a separate layer by combination in solution, either alone or with a suitable binder, followed by application with a doctor blade. Alternatively, the composition can be homogeneously blended with the film polymer, such as by forming a solution of both polymer and composition; or the composition can be blended with melted polymer. The homogeneous blend can then be cast as a film (if the solution approach

is adopted), or melted into a film, such as by use of heated platens. Many carrier matrices lend themselves to reagent application using spraying and printing techniques, such as ink jet printing.

In a preferred embodiment, filter paper is wetted with a solution or suspension of the polyelectrolyte salt in water or other convenient excipient and then dried. The polyelectrolyte-bearing filter paper is subsequently incorporated with the desired indicator means. Typically, the paper is wetted with a solution of a pH-sensitive chromogenic indicator (such as bromothymol blue) im methanol or other suitable solvent such as ethanol, N,N-dimethyl-formamide, or dimethyl sulfoxide, and subsequently dried. Alternatively, a one-dip method can be used whereby the polyelectrolyte and indicator means are simultaneously present in the initial solution or suspension.

8.3 Preparation of a Dip-and-Read Device

As indicated above, the reagent-bearing carrier matrix can be mounted on a backing material if desired. The test device, in a preferred embodiment, thus comprises a filter paper carrier matrix incorporated with the polyelectrolyte salt and an indicator means, the matrix being affixed to one end of an elongated piece of transparent polystyrene film, the other end serving as a handle. The matrix is secured to the film by any suitable means, for example by using double-faced adhesive tape (Double Stick® available from 3M Company). In use, such a device is held by the free end of the polystyrene film backing material and the matrix end is immersed into the test sample (e.g., urine) and quickly removed. Any color formation or other detectable response is observed after a predetermined time and compared with a color reference standard corresponding to responses to known solution ionic strengths or specific gravities.

9. Reference standard

The particular reference standard employed depends on whether the test means is used by itself or incorporated with a carrier matrix, and depends as well on the particular indicator means employed. Thus, if the polyelectrolyte salt is added directly to the test sample and the indicator means is a pH meter, a reference standard can be devised by adding a standard weight of polyelectrolyte salt to a standard volume of a solution of known ionic strength. The pH before and after polyelectrolyte salt addition is recorded using the pH meter. This procedure is followed for a series of solutions having differing known ionic strengths. To determine the ionic strength of an unknown test sample, the same procedure is followed and the pH change compared with those of the known solutions.

Where a test device comprising a carrier matrix containing polyelectrolyte salt and a colorometric pH indicator is employed, a reference standard can comprise a series of color blocks depicting the color developed by the carrier matrix after a predetermined time in response to solutions of known ionic strengths. When testing an unknown sample, the carrier matrix of a test device is immersed in the sample, removed, and observed for the appearance of or change in color after the predetermined time. The carrier matrix is at that time compared with the reference standard color blocks to ascertain the ionic strength or specific gravity of the sample.

10. Examples

The following non-limiting Examples are provided to further assist the reader in making and using the present invention. Thus, preferred embodiments are described and analyzed.

10.1 Preparation of Polyelectrolyte Salts

A series of experiments was conducted to prepare salts of various weakly acidic polyelectrolytes and quaternary ammonium hydroxides. The polyelectrolytes employed were maleic acid/vinylmethyl ether copolymer (Gantrez® AN—119 obtained from GAF Corporation), and maleic acid/ethylene copolymer (PMAET). The quaternary ammonium hydroxides used were tetramethylammonium hydroxide, tetrabutylammonium hydroxide, tetraethylammonium hydroxide, tributylmethylammonium hydroxide, trimethyl-2-hydroxyethylammonium hydroxide, and benzyltrimethylammonium hydroxide.

A standard solution of the particular polyelectrolyte polymer to be studied was prepared, and for each experiment two aliquots were employed. To one was added a measured amount of sodium chloride. The other comprised a solution of the polyelectrolyte in distilled water. To each aliquot was added a predetermined amount of a particular quarternary ammonium hydroxide sufficient to effect 75% substitution of the polyelectrolyte carboxyl groups. In calculating the amount of hydroxide to add, it was assumed that each of the polymers comprised alternating groups of the respective comonomers.

For each of the above-mentioned polyelectrolyte polymers, a set of solutions of 25 milliliters (ml) each was prepared in distilled water. The first solution contained sufficient copolymer to yield 0.015 moles per liter of carboxyl groups. To one of the solutions was added sufficient sodium chloride to make the solution 1.1M NaCl. To each of the solutions was added a quantity of the respective quaternary ammonium hydroxide to correspond to 0.0113 moles per liter of the corresponding quaternary ammonium ion. The exact concentrations and amounts are listed in Table I.

TABLE I

| Polyelectrolyte Solution | | Quaternary Ammonium Hydroxide | | |
|---|---|---|---|---|
| Type | Amount | Type | Amount | % Polyelectrolyte Neutralization |
| Gantrez® AN 119 | 25 ml at 2.34 g/dl in water | Tetramethylammonium hydroxide·$5H_2O$ | 1.013 g | 75 |
| Gantrez® AN 119 | 25 ml at 2.34 g/dl in water | Tetrabutylammonium hydroxide (40 g/dl in water) | 3.66 ml | 75 |
| Gantrez® AN 119 | 25 ml at 2.34 g/dl in water | Tetraethylammonium hydroxide (20 g/dl in water) | 4.14 ml | 75 |
| Gantrez® AN 119 | 25 ml at 2.34 g/dl in water | Tributylmethylammonium hydroxide (40 g/dl in water | 3.06 ml | 75 |
| Gantrez® AN 119 | 25 ml at 2.34 g/dl in water | 2-hydroxyethyltrimethylammonium hydroxide (50 g/dl in water) | 1.37 ml | 75 |
| Gantrez® AN 119 | 25 ml at 2.34 g/dl in water | Benzyltrimethylammonium hydroxide (40 g/dl in water) | 2.36 ml | 75 |
| PMAET | 25 ml of 2 g/dl in water | Tetramethylammonium hydroxide (28.6 g/dl in water) | 3.75 ml | 75 |
| PMAET | 25 ml of g/dl in water | Tetrabutylammonium hydroxide (40 g/dl in water | 3.83 ml | 75 |

10.2 Preparation of the Test Device

A test device sensitive to ionic strength or specific gravity was prepared by incorporating into filter paper a solution containing Gantrez® AN—119 which had been titrated with a quaternary ammonium hydroxide, and the pH indicator bromoethyl blue.

Into a beaker was placed 100 milliliters of a solution of 2.34 grams per deciliter (g/dl) of Gantrez® AN—119 and 0.1 grams per deciliter of bromothymol blue. This solution was titrated to a pH of 9.5± 0.02 with tetramethylammonium hydroxide.

A piece of Eaton & Dikeman No. 204 filter paper was dipped into the solution, removed and dried in a air oven at about 50°C. Squares of the impregnated dried filter paper were mounted at the end of small rectangular strips of polystyrene film measuring about 25 millimeters by about 1.01.6 millimeters (4 inches). Thus, the polystyrene film strip had a 25 millimeter square of impregnated paper mounted at one end, the other end serving as a convenient handle. The filter paper squares were mounted using double-faced adhesive tape (Double-Stick® obtained from 3M Company).

Test devices were similarly prepared using a polyelectrolyte salt prepared from Gantrez® AN-119 and tributylmethylammonium hydroxide. A solution was prepared comprising 29.3 milliliters of an aqueous solution of Gantrez® AN—119 having a concentration of 4 grams of polymer per 100 milliliters. To this was added 7.2 milliliters of an aqueous solution of tributylmethylammonium hydroxide at a concentration of 40 grams per 100 milliliters. To this mixture was added 0.05 grams (g) of bromothymol blue and 0.016 grams methyl red. The mixture was then diluted with distilled water to a final volume of 50 milliliters. The pH of this solution was measured to be 10.30 (10.26 after standing for one hour).

The solution was then used to impregnate a piece of Eaton & Dikeman filter paper as above. The filter paper was similarly dried and mounted on a piece of polystyrene film.

The test devices prepared in these experiments were found to be useful in measuring the ionic strength or specific gravity of aqueous solutions containing varying concentrations of sodium chloride.

10.3 Evaluation of the Test Device

A series of experiments was conducted in order to determine the efficacy of the test device of the present invention in determining specific gravity. The device was compared to a presently commercially available test device prepared in conformity with the claims of U.S. Patent No. 4,318,709, Falb, *et al.* The data from these experiments can be found in Figure I.

The devices of the present invention which were used in this experiment were prepared as described in section 10.2, *supra.* Instead of mounting the impregnated dried filter paper onto polystyrene strips, however, squares measuring 1 centimeter on a side were cut from the impregnated filter paper and used for the present evaluation.

In order to provide a comparison with the present invention, a test device was prepared which corresponded to the presently commercially available specific gravity measuring area of N—MULTISTIX—SG®, a multiple analyte test device marketed by the Ames Division of Miles Laboratories, Inc. This impregnated filter paper was prepared from a solution containing 23.4 grams of Gantrez® AN—119 per liter of deionized water, and 1.2 grams of bromothymol blue per liter. An aliquot of this solution was titrated with NaOH until the resultant solution pH was 7.75 as measured with a standard pH electrode and an Orion Model 701 digital pH meter. A strip of filter paper (Eaton & Dikeman No. 204) was immersed in the partially titrated aliquot and subsequently dried. After drying, the filter paper was mounted on a polystyrene film using double-faced adhesive tape (Double-Stick® obtained from 3M Company). The resultant test devices each comprised a strip of polystyrene film at one end of which was mounted a 1 centimeter square of the impregnated filter paper.

In order to compare the present invention with the NaOH-titrated Gantrez®, a series of sodium chloride solutions was prepared each having a known concentration of sodium chloride. Each of the test devices was dipped in one of the sodium chloride solutions and the surface pH of the impregnated filter paper was measured using a flat-surface Polymer-body Combination Electrode 13—639—83 obtained from Fisher Scientific Company. The pH value was recorded 60 seconds after removal of the strip from a sample solution. The concentrations and specific gravities of the sodium chloride solutions, as well as the surface pH of the respective devices following dipping into the sodium chloride solutions are recorded in Table II.

TABLE II

| NaCL Solution | | Surface pH of Test Device | | ΔpH Between Specific Gravity Levels | |
|---|---|---|---|---|---|
| Sp. Gr. | Con. (M) | Prior Device | Present Invention | Prior Device | Present Invention |
| 1.000 | 0.000 | 7.78 | 9.21 | 0.62 | 1.12 |
| 1.005 | 0.120 | 7.16 | 8.09 | 0.18 | 0.32 |
| 1.010 | 0.241 | 6.98 | 7.77 | 0.11 | 0.20 |
| 1.015 | 0.364 | 6.87 | 7.57 | 0.11 | 0.21 |
| 1.020 | 0.488 | 6.78 | 7.36 | 0.10 | 0.21 |
| 1.025 | 0.613 | 6.68 | 7.15 | 0.07 | 0.14 |
| 1.030 | 0.737 | 6.61 | 7.01 | | |

This data is plotted graphically in Figure I and shows that with the present invention a marked departure in the change in pH with respect to specific gravity occurs, whereas similar changes with the prior art device were not nearly as pronounced.

Yet another useful tool for comparison in the relative change in pH between varying specific gravity levels (ΔpH). Since the pH indicator response (or color change) is at least approximately proportional to ΔpH, it is clear that the greater the pH change for a particular device from one specific gravity level to another, the greater the color change will be, and the more the accuracy of the test will be enhanced.

ΔpH data from Table II has been plotted in Figure 2 to provide a comparison of the present invention with prior art device as prepared above (i.e., the specific gravity test area of N—MULTISTIX®—SG). Figure II shows that the present invention affords from 80% to 130% greater ΔpH with the present invention, thereby affording a dramatic increase in color response over the state-of-the-art device prior to the present invention.

**Claims**

1. A test means for determining the ionic strength or specific gravity of an aqueous test sample, wherein said test means comprises a weakly acidic polyelectrolyte polymer, and a pH-indicator means capable of producing a detectable response to ion exchange between said polyelectrolyte and said sample, characterized in that said polyelectrolyte is a salt of a weakly acidic polyelectrolyte polymer wherein at least one of the carboxyl groups of the polymer is in the form of the salt of a quaternary ammonium ion having the structure

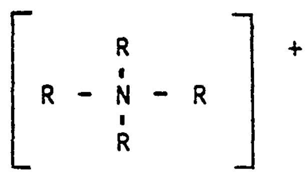

in which the R substituents, same or different, are hydrogen, 2-hydroxyethyl, benzyl, lower alkyl having one to six carbon atoms or aryl having one or more six-membered ring systems, with the proviso that at least one of R is other than hydrogen.

2. A test means according to Claim 1, characterized in that at least 50 percent of the carboxyl groups of the polymer are in the form of the salt of the quaternary ammonium ion.

3. A test means according to Claim 2, characterized in that 65 to 95 percent, preferably 90 percent, of the carboxyl groups of the polymer are in the form of the salt of the quaternary ammonium ion.

4. A test means according to any of claims 1 to 3, characterized in that the polyelectrolyte polymer is poly(acrylic acid), poly(maleic acid), maleic acid-vinyl-methyl ether copolymer, poly(methacrylic acid), or styrene-maleic acid copolymer, preferably maleic acid-vinyl-methyl ether copolymer.

5. A test means according to any of claims 1—4, characterized in that the R substituents, same of different are hydrogen, methyl, propyl, butyl, phenyl, benzyl, or 2-hydroxyethyl.

6. A test means according to Claim 5, characterized in that the ammonium ion is tetramethylammonium, tetraethylammonium, tetrabutylammonium, tetrapropylammonium, tributylmethylammonium, phenyltrimethylammonium, benzyltrimethylammonium, or 2-hydroxyethyltrimethylammonium, preferably tributylmethylammonium.

7. A test means according to any of Claims 1 to 6, characterized in that the said pH indicator substance, is bromothymol blue.

8. A test device for determining the ionic strength or specific gravity of an aqueous test sample, characterized in that it comprises a carrier matrix incorporated with the test means of any one of Claims 1 to 7.

9. A method for determining the ionic strength or specific gravity of an aqueous test sample, the method comprising contacting the sample with the test means of any of Claims 1 to 7 or the test device of Claim 8 and observing a detectable response.

## Patentansprüche

1. Testeinrichtung für die Bestimmung der Ionenstärke oder des spezifischen Gewichtes einer wässrigen Testprobe, wobei die Testeinrichtung ein schwach saures Polyelektrolyt-Polymer und eine pH-Indikatoreinrichtung, die in der Lage ist, eine nachweisbare Ansprechung auf Ionenaustausch zwischen dem Polyelektrolyten und der Probe auszubilden, umfasst, dadurch gekennzeichnet, dass der Polyelektrolyt ein Salz eines schwach sauren Polyelektrolyt-Polymers ist, in welchem wenigstens eine der Carboxylgruppen des Polymers in Form des Salzes eines quaternären Ammoniumions der Struktur

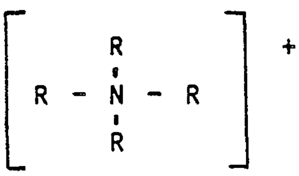

vorliegt, worin die R-Substituenten, die gleich oder verschieden sein können, Wasserstoff, 2-Hydroxyethyl, Benzyl, Niedrigalkyl mit 1 bis 6 Kohlenstoffatomen oder Aryl mit ein oder mehreren 6-gliedrigen Ringsystemen bedeutet, mit der Bedingung, dass wenigstens einer der Reste R nicht Wasserstoff ist.

2. Testeinrichtung gemäss Anspruch 1, dadurch gekennzeichnet, dass wenigstens 50% der Carboxylgruppen des Polymers in Form des Salzes des quaternären Ammoniumions vorliegen.

3. Testeinrichtung gemäss Anspruch 2, dadurch gekennzeichnet, dass 65 bis 95%, vorzugsweise 90%, der Carboxylgruppen des Polymers in Form des Salzes des quaternären Ammoniumions vorliegen.

4. Testeinrichtung gemäss einem der Ansprüche 1 bis 3, dadurch gekennzeichnet, dass das Polyelektrolyt-Polymer Polyacrylsäure, Polymaleinsäure, Maleinsäure-Vinylmethylether-Copolymer, Polymethacrylsäure oder Styrol-Maleinsäure-Copolymer, vorzugsweise Maleinsäure-Vinylmethylether-Copolymer, ist.

5. Testeinrichtung gemäss einem der Ansprüche 1 bis 4, dadurch gekennzeichnet, dass die R-Substituenten, die gleich oder verschieden sein können, Wasserstoff, Methyl, Ethyl, Propyl, Butyl, Phenyl, Benzyl oder 2-Hydroxyethylen sind.

6. Testeinrichtung gemäss Anspruch 5, dadurch gekennzeichnet, dass das Ammoniumion Tetramethylammonium, Tetraethylammonium, Tetrabutylammonium, Tetrapropylammonium, Dibutylmethylammonium, Phenyltrimethylammonium, Benzyltrimethylammonium oder 2-Hydroxyethyltrimethylammonium, vorzugsweise Tributylmethylammonium, ist.

7. Testeinrichtung gemäss einem der Ansprüche 1 bis 6, dadurch gekennzeichnet, dass die pH-Indikatorsubstanz Bromthymol Blau ist.

8. Testvorrichtung zur Bestimmung der Ionenstärke oder des spezifischen Gewichtes einer wässrigen Testprobe, dadurch gekennzeichnet, dass sie eine Trägermatrix, in welche die Testeinrichtung gemäss einem der Ansprüche 1 bis 7 inkorporiert ist, umfasst.

9. Methode zur Bestimmung der Ionenstärke oder des spezifischen Gewichtes einer wässrigen Testprobe, wobei die Methode, das Kontaktieren der Probe mit der Testeinrichtung gemäss einem der Ansprüche 1 bis 7 oder der Testvorrichtung gemäss Anspruch 8 und das Beobachten einer nachweisbaren Ansprechung umfasst.

## Revendications

1. Composition analytique pour la détermination de la force ionique ou de la densité d'un échantillon analytique aqueux, ladite composition comprenant un polymère polyélectrolytique faiblement acide et un indicateur de pH capable de déclencher une réponse détectable à l'échange d'ions entre le polyélectrolyte et l'échantillon, caractérisée en ce que le polyélectrolyte est un sel d'un polymère polyélectrolytique

faiblement acide dans lequel au moins l'un des groupes carboxyle du polymère est sous la forme du sel d'un ion ammonium quaternaire répondant à la formule

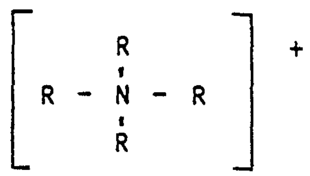

dans laquelle les substituants R, identiques ou différents, représentent de l'hydrogène, une groupe 2-hydroxyéthyle, benzyle, alkyle inférieur ayant 1 à 6 atomes de carbone ou aryle ayant un ou plusieurs systèmes cycliques hexagonaux, sous réserve qu'au moins l'un des substituants R représente autre chose que de l'hydrogène.

2. Composition analytique suivant la revendication 1, caractérisée en ce qu'au moins 50% des groupes carboxyle du polymère sont sous la forme du sel de l'ion ammonium quaternaire.

3. Composition analytique suivant la revendication 2, caractérisée en ce que 65 à 95%, de préférence 90%, des groupes carboxyle du polymère sont sous la forme du sel de l'ion ammonium quaternaire.

4. Composition analytique suivant l'une quelconque des revendications 1 à 3, caractérisée en ce que le polymère polyélectrolytique est un poly(acide acrylique), un polyacide maléique), un copolymère d'acide maléique et d'éther de vinyle et de méthyle, un poly (acide méthacrylique) ou un copolymère de styrène et d'acide maléique, de préférence un copolymère d'acide maléique et d'éther de vinyle et de méthyle.

5. Composition analytique suivant l'une quelconque des revendications 1 à 4, caractérisée en ce que les substituants R, identiques ou différents, sont de l'hydrogène, un groupe méthyle, éthyle, propyle, butyle, phényle, benzyle ou 2-hydroxyéthyle.

6. Composition analytique suivant la revendication 5, caractérisée en ce que l'ion ammonium est un ion tétraméthylammonium, tétraéthylammonium, tétrabutylammonium, tétrapropylammonium, tributylméthylammonium, phényltriméthylammonium, benzyltriméthylammonium ou 2-hydroxyéthyltriméthylammonium, de préférence tributylméthylammonium.

7. Composition analytique suivant l'une quelconque des revendications 1 à 6, caractérisée en ce que la substance indicatrice de pH est le bleu de bromothymol.

8. Dispositif analytique pour la détermination de la force ionique ou de la densité d'un échantillon aqueux à analyser, caractérisé en ce qu'il comprend une matrice de support dans laquelle est incorporée la composition analytique suivant l'une quelconque des revendications 1 à 7.

9. Méthode de détermination de la force ionique ou de la densité d'un échantillon aqueux à analyser, consistant à mettre en contact l'échantillon avec une composition analytique suivant l'une quelconque des revendications 1 à 7, ou avec le dispositif analytique de la revendication 8 et à observer une réponse détectable.

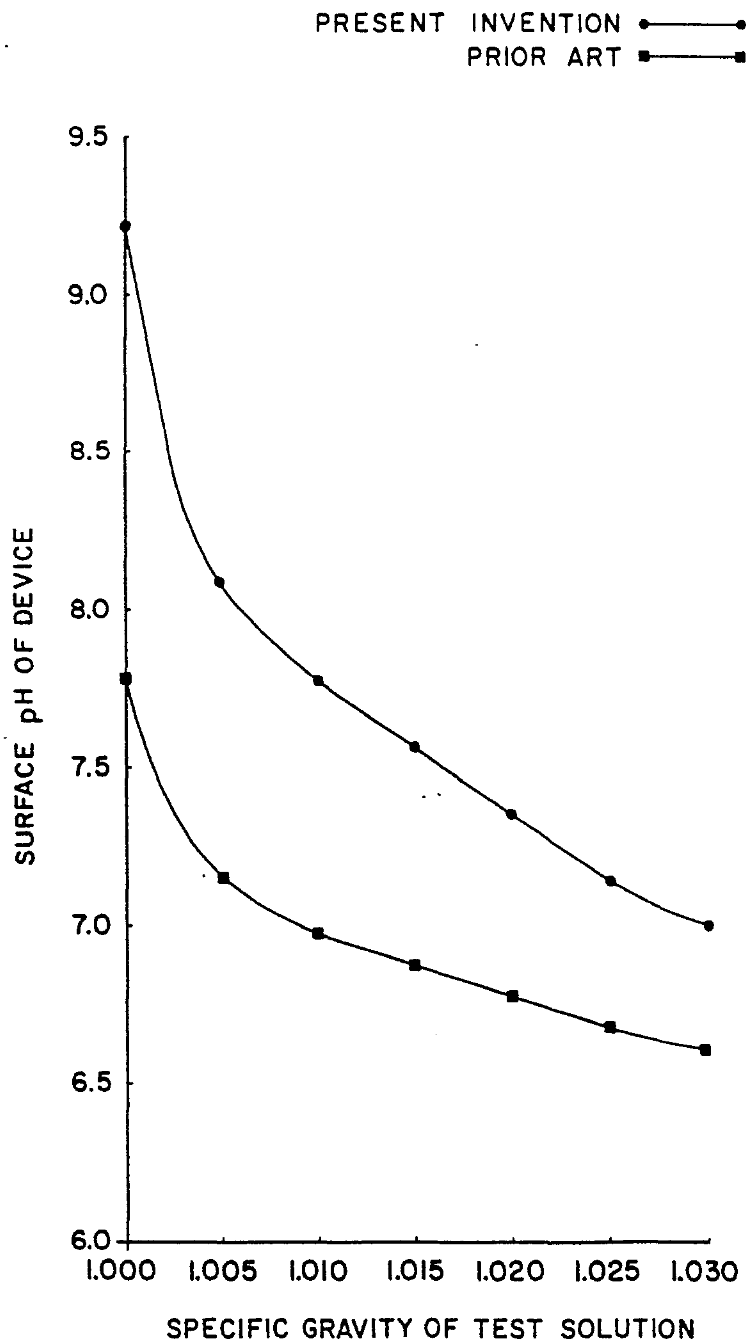
PRESENT INVENTION
PRIOR ART
9.5
9.0
8.5
8.0
7.5
7.0
6.5
6.0
SURFACE pH OF DEVICE
1.000
1.005
1.010
1.015
1.020
1.025
1.030
SPECIFIC GRAVITY OF TEST SOLUTION

FIG. 1

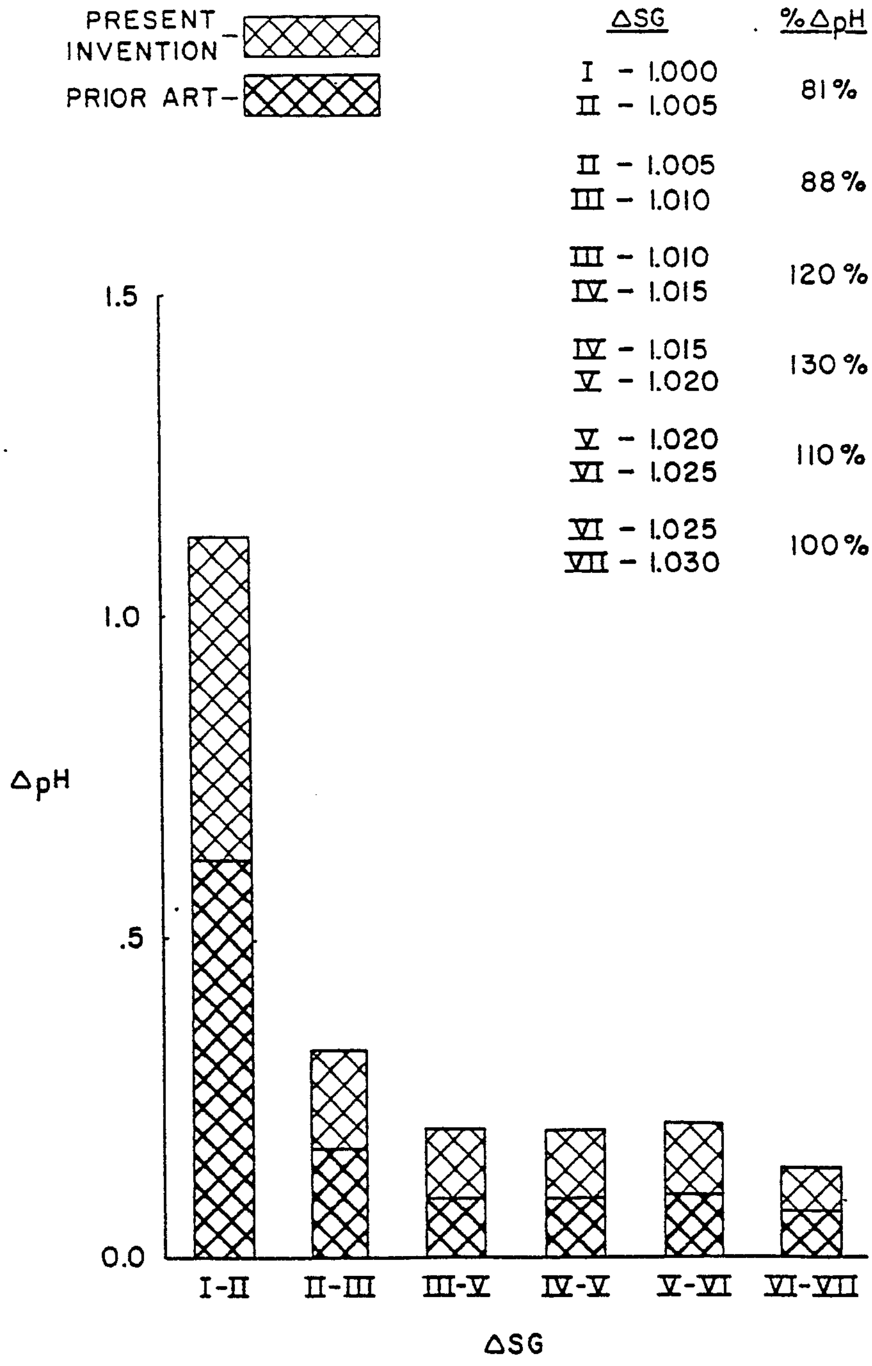
PRESENT INVENTION
PRIOR ART
ΔSG
%ΔpH
I - 1.000
II - 1.005
81%
II - 1.005
III - 1.010
88%
III - 1.010
IV - 1.015
120%
IV - 1.015
V - 1.020
130%
V - 1.020
VI - 1.025
110%
VI - 1.025
VII - 1.030
100%
1.5
1.0
ΔpH
.5
0.0
I-II
II-III
III-V
IV-V
V-VI
VI-VII
ΔSG

FIG. 2